Europäisches Patentamt

European Patent Office

Office européen des brevets

(19)

(11) Publication number : **0 465 364 A1**

(12) ## EUROPEAN PATENT APPLICATION

(21) Application number : **91401839.5**

(22) Date of filing : **03.07.91**

(51) Int. Cl.⁵ : **A23L 1/18, A23L 1/308,
A23L 1/03**

(30) Priority : **03.07.90 JP 176643/90**

(43) Date of publication of application :
**08.01.92 Bulletin 92/02**

(84) Designated Contracting States :
**BE DE FR GB IT NL SE**

(71) Applicant : **TERUMO KABUSHIKI KAISHA
No. 44-1, Hatagaya 2-chome, Shibuya-ku
Tokyo 151 (JP)**

(72) Inventor : **Watanabe, Makoto
c/o Terumo K. K., 1727-1, Tsukijiarai,
Showa-cho
Nakakoma-gun, Yamanashi-ken (JP)**

(74) Representative : **Gillard, Marie-Louise et al
Cabinet Beau de Loménie 55, Rue
d'Amsterdam
F-75008 Paris (FR)**

(54) **Antiobesity food and method for production thereof.**

(57) An antiobesity food, characterized by comprising a gelatinized starchy material and a fatty acid compound bounded thereto and possessing a bulk density in the range of from 0.1 to 0.3 g/cm³.

EP 0 465 364 A1

Field of the Invention:

This invention relates to an antiobesity food and a method for the production thereof. More particularly, this invention relates to an antiobesity food which is digested and absorbed at a slow rate and, on being ingested by a user, excites in the user's stomach or gaster the sensation of moderate fullness and a method for the production thereof.

In recent years, the patients of obesity due to enhancement of eating habits are sharply increasing in advanced societies. Persons of a high level of obesity are two to three times as susceptible of diabetes, atherosclerosis, cardiopathy, etc. Such diseases as gout, postoperative complications, cholelithiasis, lumbago, and hepatopathy which are associated with obesity are growing incessantly in prevalence. Thus, the obesity has come to pose itself a serious problem in social health.

Description of the Prior Art:

The practice of decreasing the caloric intake has been heretofore regarded as a worthwhile measure to cure and prevent the obesity. Since this practice compels the patient to feel the sensation of hunger and even starvation and discourages him from continuing the painful chore, however, it is usually performed in conjunction with one of the following methods.

One of the methods consists in appreciably narrowing the inner volume of the patient's stomach by setting a balloon fast inside the stomach or filling up the greater part of the stomach so that a small food intake may impart the stimulus of mechanical expansion to the stomach. The method of this nature, however, is undesirable because it is a permanent measure necessitating a surgical operation and possibly entailing secondary effects.

Another method consists in causing the patient to eat an extender such as dietary fibers and an adhesion enhancer optionally as mixed with other food. This method aims to lower the caloric value of food per unit weight by utilizing the nature that dietary fibers are non-digestive. Since the dietary fibers have unpleasant taste and palatability, they have the disadvantage that they cannot be easily ingested by themselves in a large amount and, even when used in conjunction with meal, they seriously impair the taste and palatability of the meal in most cases. Further, generous injection of dietary fibers is undesirable because it prevents the absorption of other beneficial nutrients and entails such secondary effects as diarrhea and constipation.

It has been recently demonstrated that carbohydrates which are digestively absorbed slowly are not closely associable with obesity as compared with carbohydrates which are digestively absorbed rapidly [Jenkins, D. J. A., et al., Am. J. Clin. Nutr. 34 1981, pp. 362-366]. It is, therefore, logically concluded that effective prevention and alleviation of obesity can be attained by using food containing carbohydrates of slow digestive absorption instead of resorting to the practice of observing a low caloric intake.

Further, the use of this food checks the otherwise possible sharp increase of the postprandial blood sugar content [Jenkins, D. J. A., et al.: The Diabetic Diet, Dietary Carbohydrate and Differences in Digestibility, Diabetologia, 23: 477-484 (1982): Collier, et al.: Effect of co-ingestion of fat on the metabolic responses to slowly and rapidly absorbed carbohydrates, Diabetologia, 25: 50-54 (1984)]. It is, therefore, inferred that the use of this food facilitates management of the morbidity and nutrition of a patient of diabetes.

As carbohydrates of slow digestive absorption, the so-called high-amylose corn starch prepared from amylomaze and various carbohydrates cooked in combination with a large volume of oil or fat have been known heretofore to the art. The former carbohydrate finds no appreciable utility because it is useful only in a limited range of applications and is deficient in taste and texture. The latter carbohydrates are not effective in combating diabetes because they bring about an addition to the caloric intake.

None of the materials for food heretofore known to the art allows slow digestive absorption, produces taste and palatability equivalent to those of ordinary starch, and finds extensive utility.

An object of this invention, therefore, is to provide a novel antiobesity food and a method for the production thereof. A further object of this invention is to provide an antiobesity food which is digested and absorbed at a slow rate and, on being ingested by a user, excites in the user's stomach or gaster the sensation of moderate fullness and a method capable of easily producing the antiobesity food.

## SUMMARY OF THE INVENTION

The antiobesity food of this invention which accomplishes the objects described above is characterized by comprising a gelatinized starchy material which possesses an amylose content in the range of about 25 to about 60% by weight and a fatty acid compound bound thereto and possessing a bulk density in the range of from about 0.1 to about 0.3 g/cm3.

The antiobesity food of this invention is desired to have a bulk density preferably in the range of from about 0.1 to about 0.2 g/cm3. The fatty acid compound is desired to be an ester of a fatty acid of 8 to 22 carbon atoms with a polyhydric alcohol.

The antiobesity food described above can be produced by preparing a raw material containing at least a starchy material which possesses an amylose content in the range of about 25 to about 60% by weight and a fatty acid compound and treating this raw material by the use of an extruder while simultaneously heating the raw material to a temperature above the level at which the starchy material can be gelatinized.

In the method for the production of the antiobesity food of this invention, the simultaneous heat treating process performed in the extruder is desired to include an action of adding water to the raw material for the sake of affording a desired product.

## DETAILED DESCRIPTION OF THE INVENTION

The present inventor performed a diligent study in search of a food possessing an antiobesity property, with a primary interest in the retardation of digestion and absorption of the food ingested in the digestive system, to find that when a fatty acid compound is bound to a starchy substance, the digestion and absorption of the starchy substance are significantly moderated. This phenomenon may be logically explained by a postulate that the hydrophobic moiety of the amylose in the starchy substance forms a complex in combination with the fatty acid compound and, because it is difficult for the formed complex to cause a structural transformation even if the complex is subjected to add water and/or heat, the starchy substance is rendered less susceptible to the action of such digestive enzymes as amylase. The present inventors continued a study with a due consideration to the essential requirement that when what is obtained by the binding of a fatty acid compound to a starchy material is used and eaten as a food, the food should create a suitable texture, and, at the same time, exhibits prominently the antiobesity effect. The inventor has consequently found that a food comprising a gelatinized starchy substance and a fatty acid compound bound thereto, and possessing a bulk density in the range of from about 0.1 to about 0.3 g/cm3 creates a preferable texture on eating, and, at the same time, gives at a small rate of ingestion to the stomach the sensation of ample inflation. The inventor further continued a diligent study, to find that the retardation of digestion and absorption of the food due to complex formation of a fatty acid compound with a starchy material arises conspicuously when this starchy material has an amylose content in the range of from about 25 to about 60% by weight. Surprisingly, this tendency could not be explained only by the increase of the fatty acid compound amount binding to the starchy material in proportion to the increase of amylose content in the starchy material. Finally, the inventor has completed this invention as an antiobesity food very effective.

The complex formation of a fatty acid compound with a starchy material is accomplished by dispersing the fatty acid compound in a suitable solvent such as water and immersing the starchy material in the resultant dispersion. This method entails the problem of operational complexity because the treatment of the raw materials requires to use the solvent in a large amount and the mixture resulting from the treatment, for the removal of the used solvent, requires to undergo the steps of solid-solution separation and drying of wet solid, for example. Further, it is relatively complicate and difficult to produce from the dried mixture prepared as described above a food possessing a desired bulk density, when necessary, by the application of heat for gelatinization of the starchy material contained in the dried mixture. In the light of this fact, the present inventors further continued a study in search of a method for easily and efficiently producing the antiobesity food possessing the quality described above, to learn that by placing the starchy material and the fatty acid compound in an extruder and treating them therein under simultaneous application of heat and pressure, the complex formation of the fatty acid compound to the thermally gelatinized starchy material is easily attained and the control of the bulk density of the resultant mixture is effected with ease. The method for producing an expanded food by the simultaneous application of heat and pressure through the aid of an extruder has been already known in the art as disclosed in Japanese Patent Unexamined Disclosures SHO 61(1986)-274,659, SHO 63(1988)-226,246, and HEI 1(1989)-252,267. The idea of utilizing the procedure of simultaneous application of heat and pressure for effecting the complex formation of a fatty acid compound to a starchy substance and the retardation of the speeds of digestion and absorption has never been known to the art and has been brought to light for the first time by the present inventors. Incidentally, Japanese Patent Unexamined Disclosure HEI 1 (1989)-252267 discloses an idea of adding a fatty acid ester to a starch substance. In this case, the fatty acid ester is added solely as an emulsifier, and in a very small amount at that. Thus, this fatty acid ester is essentially different from the fatty acid compound which is added for the purpose of retarding the speeds of digestion and absorption as contemplated by the present invention.

The expression "slow speeds of digestion and absorption" as used herein refers to the decomposition caused by amylase, for example, to an extent of not more than about 95%, preferably not more than about 85% of ordinary starch, namely, untreated starch. The antiobesity food of the present invention can be used not merely for human beings but equally for animals other than man.

Now, the present invention will be described in detail below with reference to preferred embodiments thereof.

The antiobesity food of this invention comprises a gelatinized starchy material which possess an amylose content in the range of about 25 to about 60% by weight and a fatty acid compound bound to the starchy material.

The starchy material to be used as a raw material for the antiobesity food of the present invention is adapted to possess an amylose content in the range of from about 25 to about 60% by weight, preferably from about 30 to about 50% by weight.

As universally known, starch consists of the two components, i.e. amylose and amylopectin. The starch originating in a cereal such as, for example, the starch prepared from rice, corn, barley, rye, oat, maize, potato, sweet potato, or tapioca invariably has an amylose content of less than 25%. The reason for the higher amylose content in the starchy material to be used in the present invention than in the staple starch originating in the cereal is that in the retardation of digestion and absorption by the modifier to be described more specifically hereinbelow, the use of this modifier in a small amount suffices to effect fully satisfactory modification of this starchy material of the higher amylose content and, as a result, the otherwise possibility of the starchy material's flavor and texture being impaired by the use of the modifier in a large amount is eliminated. If the starchy material has an amylose content exceeding 60% by weight, there arises the possibility that the starchy material's own flavor and texture will be notably impaired and the food's digestion and absorption will be unduly curbed by the modifier as well.

The starchy material having an amylose content in the range of from about 25 to about 60% by weight as described above may be a starch originating in a cereal, a physicochemically or biologically synthesized starch, a crude raw material for such a starch, or a processed product of such a starch. The starches of high amylose contents which are relatively readily available at present are the so-called high-amylose corn starch ( amylose content about 70%) prepared from maize of the amylomaize species and pure amylase ( amylose content 100% ). Thus, the starchy material which is obtained by mixing a starch of such a high amylose content as described above, particularly the high-amylose corn starch, with the aforementioned staple starch originating in a cereal in proportions-such that the resultant mixture acquires an amylose content in the aforementioned range of from about 25 to about 60% by weight is advantageously used in this invention.

The fatty acid compounds which are usable herein for the bonding with the starchy material as described above include free fatty acids, fatty acid salts, and fatty acid esters. These fatty acid compounds can by used more desirably when they possess a hydrophobic alkyl (fatty acid chain) moiety available for the formation of a complex with the starch mentioned above and a hydrophilic moiety, for example, hydroxyl group, available for efficient contact with the starch. In this respect, the fatty acid esters are preferred over the other fatty acid compounds mentioned above. For the reason given above, those fatty acid compounds which are destitute of such hydrophilic group as triacylglycerol cannot be used in this invention. The term "fatty acid ester" as used herein means a substance formed by the bind between one or more saturated or unsaturated alkyl compounds possessing one or more carboxyl group and one ore more compound possessing one or more alcoholic hydroxyl groups (alcohol donor) through the medium of an ester bonds. The fatty acid as a component of the fatty acid compound is desired to have 8 to 22 carbon atoms. The fatty acids which answer this description include caprylic acid, peralgonic acid, capric acid, undecylic acid, lauric acid, tridecylic acid, myristic acid, pentadecylic acid, palmitic acid, heptadecylic acid, stearic acid, nonadecanoic acid, arachic acid, behenic acid, undecylenic acid, oleic acid, elaidic acid, ectoleic acid, erucic acid, brassidic acid, linolic acid, linoleic acid, and arachidonic acid, for example.

The fatty acid salts which are usable herein include sodium salts, potassium salts, magnesium salts, etc. of the fatty acids enumerated above.

The alcohol donors owned by the fatty acid esters include glycerols, propylene glycols or polypropylene glycols, saccharides such as sucrose and reduced maltose, sugar alcohols such as sorbitol, mannitol, erythritol, and arabitol, and glycerophosphoric acid, for example. As concrete examples of the fatty acid esters, there may be cited glycerin fatty acid esters such as decaglycerol monolaurate, decaglycerol monomyristate, hexaglycerol monostearate, decaglycerol monostearate, monoglycerol monostearate, decaglycerol distearate, decaglycerol tristearate, decaglycerol monooleate, decaglycerol trioleate, hexaglycerol monooleate, and decaglycerol pentaoleate, sucrose fatty acid esters such as sucrose stearate, sucrose palmitate, sucrose oleate, sucrose laurate, and sucrose behanate, sorbitan fatty acid esters such as sorbitan monolaurate, sorbitan monostearate, and sorbitan monooleate, and lecithin, and lysolecithine. The HLB (hydrophilic lipophilic balance) of such a fatty acid ester to be used in this invention may fall in any range.

Though the ratio of addition of the fatty acid compound to the starchy material which possess an amylose content in the range of about 25 to 60% by weight in the antiobesity food of this invention depends on the kinds of the starchy material and the fatty acid compound to be actually used, the amount of the fatty acid compound is desired to be in the range of from about 0.5 to about 20 parts by weight, preferably 1 to 10 parts by weight, based on 100 parts by weight of the starchy material. If the amount of the fatty acid compound to be added is less than 0.5 part by weight based on 100 parts by weight of the starchy material, there arises a fair possibility that the retardation of digestion and absorption is not sufficiently obtained even when the starchy material and the fatty acid compound are heated and mixed in an extruder as described hereinafter to attain the complex formation of the fatty acid compound to the starchy material. Conversely, if this amount exceeds 20 parts by weight, there ensues a strong possibility that the produced food is unduly deficient in flavor and texture. In case of using the starchy material which possesses an amylose content in the range of about 25 to about 60% by weight, even though the amount of the fatty acid compound used is relatively low, a good effect of quality improvement is available in the produced food, as compared with in case of using the normal starchy material which possesses an amylose content in the range of less than 25% by weight. Therefore, the antiobesity food according to this invention has great advantages in effect to the living body, in flavor and texture, and in costs.

The antiobesity food of this invention is only required to comprise at least a starchy material which possesses an amylose content in the range of about 25 to about 60% by weight and a fatty acid compound as described above. It may, when necessary, incorporate additionally therein from the nutritional aspects, proteins such as casein, soybean protein and skimmed milk or the like, mineral sources such as iron citrate and calcium carbonate or the like, and various vitamins, and from the standpoint of improving the taste and texture, oils and fats (inclusive of powdered oils and fats) such as coconut oil and lard, dietary fibers such as mannan, carrageenin, and guar gum, sweeteners such as stevia and reduced maltose, table salt, corn fiber cacao powder, various fruits, various fruit flavors and spices.

The antiobesity food of this invention is required to possess a bulk density approximately in the range of from about 0.1 to about 0.3 g/cm3, preferably about 0.1 to about 0.2 g/cm3. If the bulk density deviates from this range, the texture on eating and the sensation of fullness excited in the stomach are both short of being fully satisfactory.

The antiobesity food of this invention can be produced by kneading the starchy material having an amylose content in the range of about 25 to about 60% by weight and the fatty acid compound described above in combination with optionally selected additives under simultaneous application of heat and pressure by the use of an extruder, for example. The extruder to be used herein may be of any kind on the condition that it is capable of imparting a kneading motion to the raw material placed therein and simultaneously exerting heat and pressure thereon. Numerous kinds of single-screw, twin-screw, and multiple-screw extruders are available.

The temperature at which the raw material and additives placed in the extruder are to be processed in the production of the antiobesity food of this invention is required to be not less than 60°C, preferably desired to be in the range of from about 120°C to about 200°C. Since the processing to be performed in the extruder as described above requires not only to effect complex formation of the fatty acid compound to the starchy material in the raw material but also give rise to a food containing the starchy material in a gelatinized state, the lower limit of the temperature is defined to exceed the temperature at which the starchy material begins to be gelatinized (generally in the range of from about 60°C to about 70°C, though variable with the particular kind of starchy material to be actually used). Suitably, the pressure to be exerted during the mixture of the raw material and the extrusion of the resultant mixture from the device is in the range of from about 10 to about 200 kgf/cm², preferably from about 20 to about 150 kgf/cm².

When the raw material is to be kneaded in an extruder under simultaneous application of heat and pressure, water may be added, when necessary, to the raw material. The water thus added either promotes or aids in the mixture of the raw material, the gelatinization of the starchy material contained in the raw material, the binding of the starchy material and the fatty acid compound in the raw material, etc. The added water further affects the bulk density of the produced food. Generally, the extruded mixture possesses a large bulk density and exhibits high rigidity when the amount of the added water is large and the extruded mixture possesses a small bulk density and exhibits low rigidity when the amount of the added water is small. Though the amount of water to be added during the mixture of the raw material in the extruder under simultaneous application of heat and pressure hinges on the kinds of the raw material as the starchy material and the fatty acid compound and on the conditions of processing in the extruder, it is generally in the range of from about 2 to about 20%, preferably about 5 to about 10%, by weight based on the total amount of the starchy material and the fatty acid compound plus the additives optionally selected for incorporation into the food.

The product of extrusion resulting from kneading the raw materials and the additives in the extruder under simultaneous application of heat and pressure is subjected, when necessary, to further steps of processing such as shaping by pulverization or cutting, drying, and seasoning which substantially has no effect to the weight of a finished good, to be completed as a finished good. It may be accomplished after above procedure that the bulk density of the antiobesity food of this invention falls in the aforementioned range. Therefore, It is acceptable that just after extruding the bulk density is not in the aforementioned range but after shaping and drying it is in the range.

In the production of the antiobesity food contemplated by this invention, the adjustment of the bulk density within the prescribed range needs not rely solely on the amount of water added during the processing by the use of the extruder. This adjustment may be otherwise attained, for example, by the adjustment of the amount of the raw material to be supplied, the adjustment of the revolution number of the shafts of the extruder, or the alteration of screw pattern. As respects the method itself which is adopted for the production of the antiobesity food of this invention, it needs not be limited in any sense to the method which resorts to the use of the extruder as described above. The production may be otherwise attained, for example, by a method which comprises bringing the starchy material and the fatty acid compound into contact with each other in a suitable medium such as water and thereafter subjecting the resultant mixture to a proper heat treatment, on the condition that this method gives rise to a product which comprises a gelatinized starchy material and a fatty acid compound bound thereto and possesses a prescribed bulk density.

EXAMPLES

Now, the present invention will be described more specifically below with reference to working examples. It should be noted, however, that this invention is not limited to or by the examples so cited.

Example 1:

8.3 kg of commercially available flour having an amylose content of about 22% , and 1.7 kg of commercially available high-amylose corn starch having an amylose content of about 70% were mixed in order to prepare a starchy raw material having an amylose content of about 30%. To 10 kg of the prepared starchy raw material, 100 g of a sucrose stearate (produced by Mitsubishi Chemical Industry Co., Ltd. and marketed under product code of "S-790") was added and mixed. The resultant mixture was placed in a twin-screw extruder (produced by Kowa Kogyo K.K. and marketed under product code of "EI 45 Type") and kept stirred therein under continued introduction thereto of water added in a total amount of 2% based on the amount of the mixture. The blend was extruded from the extruder, with the pressure near the outlet fixed at 45 kgf/cm$^2$ and the temperature at 160°C. The extruded blend was cut into balls with a cutter and then left drying for five minutes in an oven kept at 170°C, to produce spheres of food sample about 7 mm in diameter. This sample was found to have a bulk density of 0.118 g/cm$^3$.

This sample was tested for digestibility with porcine pancreas α-amylase (PPA) by the following method. As a control, a sample produced by following the procedure described above, excepting the addition of sucrose stearate was omitted, was used. The digestibility of the sample of this invention with the PPA was found to be 86% of that obtained of the sample of the control.

A panel of 10 volunteers were asked to try 30 g of the sample of this example in hungry condition early in the morning, and evaluate its texture on eating and the sensation of fullness of the stomach after the elapse of five hours following the time of ingestion. The results are shown in Table 1. It is noted from this table that the sample of this example were both satisfactory in terms of texture on eating and sensation of fullness. Examples 2 to 4:

Food samples were obtained by following the procedure of Example 1, excepting the amount of water added to the twin-screw extruder was changed to 5% (Example 2), 10% (Example 3), and 20% (Example 4), respectively by weight based on the amount of the mixture of raw material. These samples were tested for bulk density and digestibility with PPA and further put to a sensory test for texture on eating and sensation of fullness. The results are shown in Table 1.

Controls 1 to 3:

Food samples were obtained by following the procedure of Example 1, except that the amount of water added to the twin-screw extruder was changed to 1.5% (Control 1), 25% (Control 2), and 30% (Control 3), respectively by weight based on the amount of the mixture of raw material. These samples were tested for bulk density and digestibility with PPA and further put to a sensory test for texture on eating and sensation of fullness. The results are shown in Table 1.

Referential example:

A food sample was obtained by following the procedure of Example 1, except that a starchy raw material having an amylose content of less than 25% was used in stead of the high amylose content raw material. Namely, the food sample was obtained by following the procedure of Example 1, except that as the starchy raw material only the commercially available flour having an amylose content of about 22% was used. These sample was tested for bulk density and digestibility with PPA and further put to a sensory test for texture on eating and sensation of fullness. The results are shown in Table 1. As a control, a sample produced by following the procedure described above, excepting the addition of sucrose stearate was omitted, was used.

Table 1

| | Bulk density (g/cm3) | Digestibility with PPA (%) | Sensation of fullness[1] | Number of panel members reporting presence of sensation of fullness | Texture on eating[2] | Number of panel members reporting satisfaction to texture on eating | Remark |
|---|---|---|---|---|---|---|---|
| Control 1 | 0.095 | 88 | Poor | 3/10 | Poor | 4/10 | Sticky to teeth |
| Example 1 | 0.108 | 86 | Fair | 6/10 | Excellent | 9/10 | |
| Example 2 | 0.129 | 85 | Excellent | 10/10 | Excellent | 10/10 | |
| Example 3 | 0.150 | 84 | Excellent | 8/10 | Excellent | 10/10 | |
| Example 4 | 0.235 | 85 | Fair | 6/10 | Fair | 6/10 | |
| Control 2 | 0.350 | 83 | Poor | 4/10 | Poor | 2/10 | Hard |
| Control 3 | 0.392 | 83 | Poor | 4/10 | Poor | 0/10 | Extremely hard |
| Referential example | 0.10 | 93 | Fair | 6/10 | Excellent | 9/10 | |

*1: As respects sensation of fullness, the rate "poor" was given to a sample which drew the report of sensation of fullness from not more than four out of the ten panel members, the rate "fair" to a sample which drew the same report from five or six of the ten panel members, and the rate "excellent" to a sample which drew the same report from at least seven of the ten panel members.

*2: As respects texture on eating, the rate "poor" was given to a sample which drew the report of satisfaction to texture on eating from not more than five of the ten panel members, the rate "fair" to a sample which drew the same report from six to eight of the ten panel members, and the rate "excellent" to a sample which drew the same report from at least nine of the ten panel members. For the samples rated as poor, the main reason for unfavorable evaluation secured from dissatisfied panel members is shown in the column headed as "Remark."

EP 0 465 364 A1

It is clearly noted from Table 1 that the food samples of Examples 1 to 4 conforming to the present invention were invariably evaluated as highly satisfactory in the sensory test as to texture on eating and sensation of fullness and invariably found in the test for digestibility with PPA as exhibiting significant declines as compared with the samples of the control, indicating that they attained retardation of digestion and absorption.

The food sample of Referential example was also evaluated in the sensory test as highly satisfactory. However, the sample of Referential example was inferior to those of Examples 1 to 4 in respect of the decrement in the PPA digestion ratio. Since each values of the PPA digestion rate is indicated as a relative value to a control, and the control for Referential example is different from the control for Examples 1 to 4, it is not able to judge at a concrete degree which sample is digested and absorbed more slowly by directly comparing the value of Referential example with those of Examples 1 to 4. But, it is easily understood that each samples of Examples 1 to 4 is digested and absorbed at a rather slow rate than that of Referential example, by concerning that each starchy raw materials used in Examples 1 to 4 is naturally excellence to the starchy raw material of Referential example in the gentleness for the digestion and absorption rate, and that the degree of decrement in the PPA digestion rate obtained in Examples 1 to 4 are greater than that of Referential example.

Example 5:

8 kg of commercially available rice powder having an amylose content of about 24% , and 2 kg of commercially available high-amylose corn starch having an amylose content of about 70% were mixed in order to prepare a starchy raw material having an amylose content of about 33%. To 10 kg of the prepared starchy raw material, 500 g of a glycerin monostearate (produced by Riken Vitamins K.K. and marketed under trademark designation of "Emulsy-MS") was added and mixed. The resultant mixture was placed in a twin-screw extruder (produced by Kowa Kogyo K.K. and marketed under product code of "EI 45 Type") and kneaded under continued introduction of water added in a total amount of 5% by weight based on the amount of the mixture. The mixture was then extruded from the extruder, with the pressure near the outlet fixed at 38 kgf/cm$^2$ and the temperature at 190°C. The extruded mixture was cut with a cutter into balls and then left drying for five minutes in an over kept at 170°C, to produce spheres of a food sample about 5 mm in diameter.

The sample of Example 5 is found by test to possess a bulk density of 0.173 g/cm$^3$.

As a control, a food sample was prepared by following the procedure described above, except that the addition of glycerin monostearate was omitted and the conditions of water addition, pressure and temperature were changed properly so as to get the same bulk density as the sample of Example 5.

In the same sensory test as performed in Example 1, the food samples were invariably evaluated as "excellent" in terms of texture on eating and sensation of fullness.

A healthy adult male volunteer was asked to ingest 30 g each of the food sample of Example 5 and the sample of the control. At fixed intervals following the time of the ingestion, bloods were extracted and tested for blood sugar. For the determination of blood sugar, a commercially available tester (produced by Eims-Sankyo K.K. and marketed under trademark designation of "Glucoster") was used. It is clearly noted from the results that when the sample of this invention was ingested, the rise of blood sugar was repressed as evinced by 75% of surface area and 79% of peak height respectively of the corresponding magnitudes exhibited by the sample of the control.

Example 6:

900 kg of commercially available flour having an amylose content of about 22% , and 100 kg of commercially available high-amylose corn starch having an amylose content of about 70% were mixed in order to prepare a starchy raw material having an amylose content of about 27%. To 10 kg of the prepared starchy raw material, 250 g of a glycerin monostearate (produced by Riken Vitamins K.K. and marketed under trademark designation of "Emulsy-MS") and 800 g of skimmed milk from the nutritional point of view and 200 g of corn fibers added thereto from the standpoint of improving texture were added and mixed. The resultant mixture was placed in a twin-screw extruder (produced by Kowa Kogyo K.K. and marketed under product code of "EI 45 Type") and kept kneaded therein under continued introduction of water added thereto in a total amount of 7% by weight based on the amount of the mixture. The produced mixture was extruded from the extruder with the pressure near the outlet fixed at 78 kgf/cm$^2$ and the temperature at 180°C. The extruded mixture was cut with a cutter into balls and then left drying for 15 minutes in a continuous fluidized-bed type drier kept at 80°C, to obtain food sample as granules of about 3 mm in diameter. This food sample was found to have a bulk density of 0.20 g/cm$^3$. In the same sensory test as performed in Example 1, this food sample was evaluated as "excellent" in terms of both texture on eating and sensation of fullness.

The determinations involved in the working examples of this invention were carried out by the following procedures.

Bulk density:

In a rectangular parallelepiped container (1 liter of inner capacity) having an inner bottom area of the square of 70 mm and a height of 204 mm and opened on the upper side, a given food sample was placed so as to reach but not protrude from the upper end of the container and was weighed. The bulk density of this food sample was computed in accordance with the following formula.

Bulk density (g/cm$^3$) = Weight of the food sample held in the container (g) / 1,000

Digestibility with porcine pancreas $\alpha$-amylase (PPA):

A given food sample was pulverized until an average particle diameter of 100 $\mu$m. A 0.5-g portion of the pulverized food sample and 49 ml of a 50 mM phosphate buffer solution (pH 6.9) added thereto were left standing for 30 minutes in a shaken constant temperature bath adjusted at 37°C. One (1) ml of an enzyme solution prepared by diluting PPA (produced by Sigma) with a phosphate buffer solution to a total amount of 50 $\mu$U/ml was added to the mixture to initiate a reaction. After the elapse of 0, 20, 40, and 60 minutes following the initiation of the reaction, two specimens each 0.2 ml in volume were extracted from the bath and placed in test tubes containing 3.8 ml of 0.1N NaOH to stop the enzymatic reaction in process therein.

The amount of reducing sugar produced by the digestion with PPA was determined by the Somogyi-Nelson method.

## Claims

1. An antiobesity food, characterized by comprising a gelatinized starchy material which possesses an amylose content in the range of about 25 to about 50% by weight and a fatty acid compound bounded thereto and possessing a bulk density in the range of from about 0.1 to about 0.3 g/cm$^3$.

2. An antiobesity food according to claim 1, wherein said gelatinized starchy material possesses an amylose content in the range of about 30 to about 50% by weight.

3. An antiobesity food according to claim 1, wherein said bulk density is in the range of about 0.1 to about 0.2 g/cm$^3$.

4. An antiobesity food according to claim 1, wherein the ratio of enzymatic digestion with amylase is lowered to not more than about 95% of said starchy material in an unaltered state.

5. An antiobesity food according to claim 1, wherein said fatty acid compound is an ester between a fatty acid of 8 to 22 carbon atoms and a polyhydric alcohol.

6. An antiobesity food according to claim 1, wherein said fatty acid compound is at least one member selected from the group consisting of glycerin fatty acid esters, sucrose fatty acid esters, sorbitan fatty acid esters, lecithin, and lysolecithin.

7. An antiobesity food according to claim 1, wherein said fatty acid compound is incorporated in an amount in the range of from about 0.5 to about 10 parts by weight, based on 100 parts by weight of said starchy material.

8. A method for the production of an antiobesity food of claim 1, characterized by preparing a raw material containing at least a starchy material which possesses an amylose content in the range of about 25 to about 60% by weight and a fatty acid compound and treating said raw material in an extruder and simultaneously heating said raw material therein at a temperature high enough to induce gelatinization of said starchy material.

9. A method according to claim 8, wherein said step of heat treatment in said extruder embraces an action of adding water to said raw material.

10. A method according to claim 8, wherein the amount of water added to the raw material is in the range of about 2 to about 20% by weight, based on the amount of raw material.

European Patent
Office

**EUROPEAN SEARCH REPORT**

Application Number

EP 91 40 1839

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 113 056 (NESTLE)<br>* claims 1,6 *<br>* page 2, line 7 - page 4, line 7 *<br>* examples 1,3,4 *<br>--- | 1-3,5-10 | A23L1/18<br>A23L1/308<br>A23L1/03 |
| Y | WORLD PATENTS INDEX<br>Derwent Publications Ltd., London, GB;<br>AN 76-59134X(31)<br>& JP-B-51 022 059 (HOHNEN OIL) 8 July 1976<br>* abstract *<br>--- | 1-3,5-10 | |
| A | R.JOWITT 'EXTRUSION COOKING TECHNOLOGY'<br>1984 , ELSEVIER , LONDON<br>* page 194 - page 195 *<br>--- | 4 | |
| A | R.B.FAST 'BREAKFAST CEREALS AND HOW THEY ARE MADE' , AMERICAN ASSOCIATION OF CEREAL CHEMISTS , ST PAUL, USA<br>* page 41 *<br>--- | 1,3 | |
| A | US-A-3 753 729 (VAN DEURSEN HARMS)<br>* claim 1 *<br>* column 1, line 56 - column 2, line 21 *<br>* column 2, line 30 - line 55 *<br>--- | 1-10 | **TECHNICAL FIELDS SEARCHED (Int. Cl.5)**<br><br>A23L |
| A,P | EP-A-0 388 319 (TERUMO)<br>* claims 1,12 *<br>* page 2, line 1 - page 2, line 39 *<br>* page 4, line 46 - page 5, line 25 *<br>* example 48 *<br>----- | 1-6 | |

The present search report has been drawn up for all claims

| Place of search | Date of completion of the search | Examiner |
|---|---|---|
| THE HAGUE | 17 OCTOBER 1991 | VUILLAMY V.M.L. |

EPO FORM 1503 03.82 (P0401)